# EUROPEAN PATENT APPLICATION

(11) **EP 1 617 221 A1**
(43) Date of publication of application: **18.01.2006**
(21) Application number: 03717707.8
(22) Date of filing: 23.04.2003
(51) Int. Cl.: G01N 33/566, G01N 33/50, G01N 33/542

(54) **METHOD FOR SCREENING SUBSTANCE WHICH CAN BIND TO RECEPTOR**

(71) Applicant: Olympus Corporation, Tokyo 151-0072 (JP)
(72) Inventor: KATO, Noriko, Hachioji-shi, Tokyo 192-0046 (JP)
(74) Representative: von Hellfeld, Axel
(86) International application number: PCT/JP2003/005188
(87) International publication number: WO 2004/095028

(57) **Abstract**

A method for screening a substance which can optimally bind to a receptor, by monomolecular fluorescent analysis, comprising a step of searching a substance which can bind to a receptor, and a step of performing optimization of a structure of the substance which can bind to the receptor obtained by the above step.

## Description

### Technical Field

The present invention relates to a method for screening a substance which can bind to a receptor. Particularly, the method of the present invention is useful in structure-activity correlation analysis for effectively finding out a ligand for a novel drug design target, and performing molecular design for drug design.

### Background Art

### (Background in drug development screening)

Drug development is to find out or create a molecule having drug efficacy, or a molecule which can be applied to treatment of a disease. During the process of drug development, screening must be performed in consideration of drug efficacy, bioavailability, *in vivo* distribution, a metabolism rate, an excretion rate, side effect and toxicity, and thus, this is a difficult work requiring much time and cost.

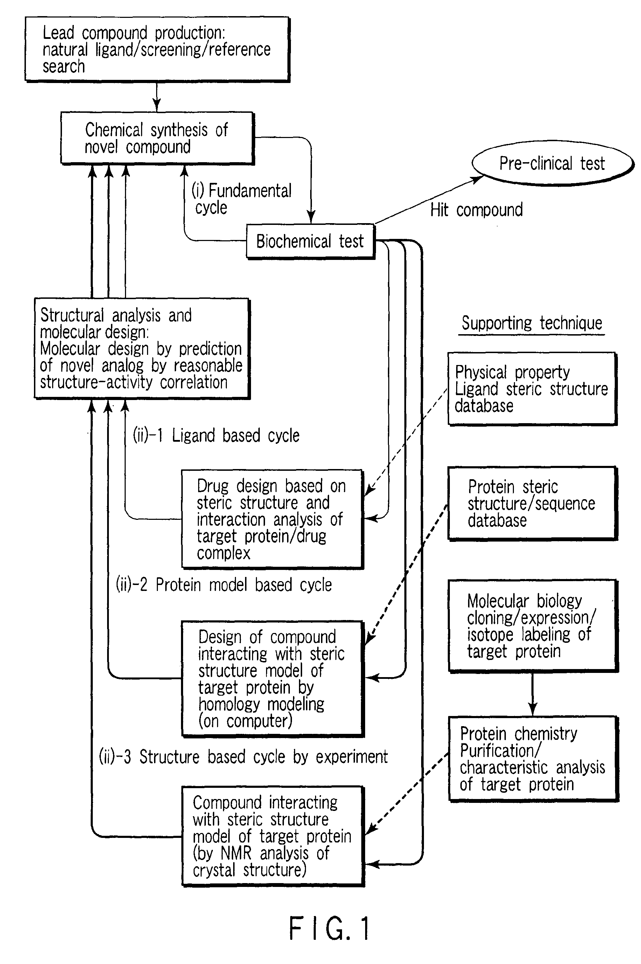

Drug design study has, roughly, two study cycles (see FIG. 1; see Greer J, Erickson JW, Baldwin JJ, Varney MD., J Med Chem 1994 Apr 15; 37(8):1035-54: Application of the three-dimensional structures of protein target molecules in structure-based drug design.). The first cycle (shown by (i) in FIG. 1) is a "cycle in which structural modification and drug efficacy assessment of a lead compound are repeated while quantitative structure-activity correlation is considered, relying on experience and intuition of a synthesis researcher". This cycle was a mainstream until 1980s. By development of high throughput screening technique due to combinatorial chemistry and robotization in recent years, a necessary time in synthesis and drug efficacy assessment has been considerably shortened, and this cycle has been developed to synthesis and screening of hundreds thousands compounds. However, whether a hit compound having sufficient activity is obtained or not greatly depends on a screening system and a compound library for performing screening, and thus a hit compound has not been obtained in some cases in this cycle.

On the other hand, since late 1980s, results of steric structural analysis of proteins have been gradually increased and, as a result, structure-based drug design (SBDD) has appeared in which drug design is experimentally performed based on steric structure and interaction analysis of a target protein/drug complex. In addition, a method of constructing a steric structural model of a target protein by homology modeling, and designing a compound which can interact with the protein by docking on a computer has been generally used. These are shown by (ii)-1 to (ii)-3 of drug design cycles in FIG. 1.

Today, with development of structural biology, steric structures of many biopolymers are revealed by X-ray crystal analysis and nuclear magnetic resonance (NMR). On the other hand, with development of structural genome science project which is a part of post genome project, over 2005 to 2010, novel steric structures of about 10,000 species of proteins have been analyzed, and they are planned to be published on database. Further, if registration of representative structures of almost all protein families on database is realized, it becomes possible to construct a steric structure from steric structures of analogous proteins on database by homology modeling, regarding any drug design target. There is a high possibility that, based on study results of structural genome science project, structure-based drug design (SBDD) will be fixed in Japan in near future.

However, important in the drug design cycle in FIG. 1 is that unless a lead compound or a candidate compound thereof is identified, all cycles are not initiated. Even when a drug design target protein is selected from genome information, and its steric structure can be immediately utilized, it is still difficult to design a lead compound as a drug only from a steric structure of a protein, and the situation that a compound binding to the protein with high affinity and high selectivity must be searched is unchanged as previously.

On the other hand, as a procedure for analyzing a steric structure, in 1996, a group of SW Fesik published "a process for designing a lead compound by combining screening of compounds by NMR and linked fragment approach", so-called SAR by NMR (Structure-activity relationship by nuclear magnetic resonance) (Reference Publication: Shuker SB, Hajduk PJ, Meadows RP, Fesik SW., Science, 1996 Nov 29, 274 (5292), 1531-4, Discovering high-affinity ligands for proteins: SAR by NMR). NMR has been recognized as one procedure for analyzing a steric structure of a protein, and has been introduced for the purpose of SBDD in each pharmaceutical company in early 1990s. Before that time, since NMR has a low sensitivity, and needs a time for experimentation, screening by NMR was not considered at all and, therefore, a great attention was paid all the more.

### (Drug design screening by NMR)

NMR is now widely applied as a method of analyzing a steric structure of a biopolymer such as a protein and a nucleic acid, as well as X-ray crystal analysis. However, application of NMR is limited to a molecule having a molecular weight of 20 to 30 thousands or lower. Generally, a molecule of 20 to 30 thousands or lower is a standard size as a unit of a functional domain to which a drug binds. However, analysis of a steric structure by NMR needs a few days to one month, and it is actually impossible to analyze steric structures one by one for the purpose of screening compounds. Even when a lead compound has been already found out, it is more effective to synthesize a candidate compound by using combinatorial chemistry and perform an assay than taking a time for steric structure analysis and drug design therefrom.

NMR analysis and its characteristic will be explained below. An advantage of NMR is that spectroscopy can be performed, and that a NMR signal sensitively reflects a local change in the state of a molecule (drug binding, structural change, change in mobility). When a protein (P) and a ligand (L) are bound to form a complex (P-L), a NMR spectrum of a complex (P-L) is different from a NMR spectrum when P and L are present separately. By using such a change in the spectrum as an index, a binding assay of a protein and a drug can be performed. Characteristics of NMR when used in random screening are as follows:
(i) NMR has a lower sensitivity as compared with spectroscopy such as UV, CD and fluorescence, and a protein having a concentration of 0.2 mM or higher is necessary. In addition, 250 to 300 µL of a sample is necessary for one time measurement.
(ii) Generally, in a protein sample of around 0.3 mM, one-dimensional NMR needs a measuring time of a few minutes, and two-dimensional NMR needs a measuring time of about 15 minutes.
(iii) Only NMR signal of a protein can be observed by utilizing ¹⁵N-¹HHSQC spectrum using a protein uniformly labeled with ¹⁵N.
(iv) When a NMR signal of a protein has been assigned, a drug binding site can be specified from a signal which has been altered at the time of drug addition.

Regarding a low measurement sensitivity of characteristic (i), as a drawback, it is necessary that both of a protein and a ligand are dissolved in water at a high concentration. In addition, regarding a characteristic (ii), a speed of an assay has a limit, and this is not suitable for high throughput. In order to speed up treatment, it is actually difficult to prepare a few of high magnetic field NMR apparatuses having a cost of a few hundreds millions per apparatus.

On the other hand, a characteristic (iii) is an advantage which is not possessed by other spectroscopies and, even when a drug is added at a high concentration (excessively), a spectrum of a protein can be measured without being hampered by a signal of the drug. Further, as an advantage deriving from characteristics (i) and (iv), since a sample concentration is high, a compound which site-specifically binds to a protein can be found out even when affinity is low (even when a dissociation constant is around 0.1 to 1 mM). In particular, a compound exhibiting only low affinity for a target protein is not regarded as a hit compound in a normal assay system, but such a compound is an important candidate compound which can have a partial structure of a lead compound in that it site-specifically binds to a protein. As compared with other assays utilizing a chemical reaction and biological responses, an advantage of NMR is that a hit compound can be screened with information of a binding site, and direct interaction between a protein and a drug can be detected.

SAR by NMR is an abbreviation of structure-activity relationship obtained by NMR, and is a procedure involving searching and optimizing a structure of a lead compound. This procedure consists of "screening of a low-molecular compound (fragment) which binds to a target protein, using NMR", and "improvement in affinity by covalently linking compounds obtained by screening via a linker (linked fragment approach)".

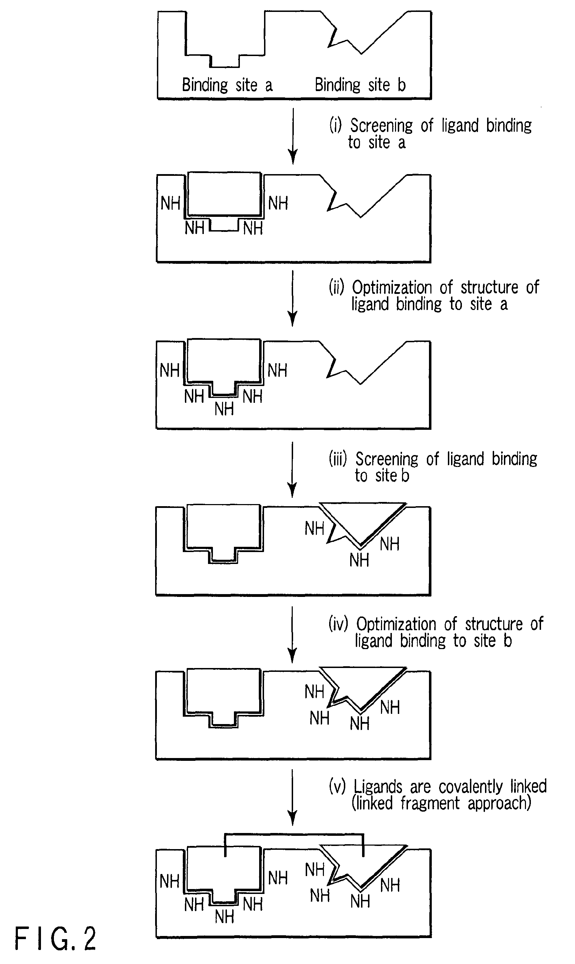

FIG. 2 shows outline of SAR by NMR. Each step of (i) to (v) of FIG. 2 will be explained.
"(i) Screening of a ligand which binds to a site a"
   Using, as an index, a change in a NMR chemical shift when a ligand is added to a solution of a target protein labeled with ¹⁵N, a compound (hit compound) which directly interacts with a particular site (binding site a) of a target protein is screened from a compound library.
"(ii) Optimization of a structure of a ligand which binds to a site a"
   A structure of a hit compound is modified, and a compound which most strongly binds to a binding site a is found out (structure-activity correlation).
"(iii) Screening of a ligand which binds to a site b"
   A hit compound which binds to a binding site b in a vicinity thereof is screened in the presence of the compound obtained in (ii), as in the step of (i).
"(iv) Optimization of a structure of a ligand which binds to a site b"
   Optimization of a structure of a hit compound is also performed on a binding site b, as in the step of (ii) (structure-activity correlation).
"(v) Ligands are covalently linked (linked fragment approach)"
   By covalently linking compounds which bind to two sites a and b, affinity for a target protein is improved (linked fragment approach).

In this procedure, a compound having a little over 200 of an average molecular weight can not respond, and measurement can not be performed also in the step of (v) unless an attention is paid so that a molecular weight dose not become too great.

There is a report in which an inhibitor of a FK506-binding protein (FKBP) was searched by the aforementioned method utilizing NMR (Shuker SB, Hajduk PJ, Meadows RP, Fesik SW., Science, 1996 Nov 29, 274 (5292), 1531-4, Discovering high-affinity ligands for proteins: SAR by NMR). In this method, FKBP uniformly labeled with ¹⁵N is used, and about 1000 compounds of library were screened. As a result, it was detected that certain two kinds of compounds have a high affinity for FKBP, when they bind to FKBP.

Screening of a compound (fragment) according to the aforementioned NMR spectroscopy has the following problems.
(1) High magnetic field NMR which is a measuring machine necessary for drug design screening has a very high price of 100 to 200 millions per machine, and a special sample tube and automatical sample changer are necessary.
(2) Since operation of the apparatus needs specialty greatly, both of a NMR technologist and a biochemist are necessary.
(3) As a sample, a large amount (200 mg or more) of a target protein labeled with ¹⁵N is necessary and, additionally, as a concentration of a sample, a high concentration of not lower than 0.2 mM is necessary. In addition, a sample having a high purity is necessary for NMR measurement, and a crudely purified material containing a target protein can not be used as a sample. Therefore, preparation of a sample is complicated, and needs a time.
(4) A measurable molecular weight of a molecule is limited to 20 to 30 thousands, and the screening can not be utilized in a protein of 20 kDa or more. Since NMR must be investigated every protein domain, the screening is not suitable for screening a drug having a binding site spanning a plurality of domains.
(5) The screening is not suitable for measuring a sample having a complicated structure such as a lipid and a glycoprotein.
(6) Unless a structure of a target protein and a compound is already known, the screening can not be utilized. Therefore, when a side chain of a compound is randomly altered, the screening can not be immediately utilized in measuring that compound.
(7) Whether a test compound has been bound to a target protein or not can be detected, but strength of a binding force can not be investigated.
(8) Since the screening needs a time for NMR measurement, it is not suitable for high throughput screening.

### Disclosure of Invention

In view of the aforementioned circumstances, an object of the present invention is to provide a novel screening method which can solve all the problems possessed by the conventional screening method such as SAR by NMR.

The present inventors found out a new method of screening simply and rapidly a substance which can specifically binds to a receptor, using a nonradioactive substance as a detection marker, which resulted in completion of the present invention. The method of the present invention solves problems possessed by the conventional screening method such as SAR by NMR at once.

That is, the present invention provides the following means.
(1) A method for screening a substance which can optimally bind to a receptor, by monomolecular fluorescent analysis, comprising:
   a step of searching a substance which can bind to a receptor; and
   a step of performing optimization of a structure of the substance which can bind to the receptor obtained by the above step.
(2) A method for screening a substance which can bind to a receptor having a first binding site and a second binding site, by monomolecular fluorescent analysis, comprising:
   a step of searching a substance which can bind to the first binding site; and
   a step of searching a substance which can bind to the second binding site, under condition where the substance which can bind to the first binding site obtained by the above step is bound to the receptor.
(3) A method for screening a substance which can optimally bind to a receptor having a first binding site and a second binding site, by monomolecular fluorescent analysis, comprising:
   a step of searching a substance which can bind to a first binding site;
   a step of performing optimization of a structure of the substance which can bind to the first binding site obtained by the above step;
   a step of searching a substance which can bind to the second binding site, under condition where the substance which can optimally bind to the first binding site obtained by the above step is bound to the receptor; and
   a step of performing optimization of a structure of the substance which can bind to the second binding site obtained by the above step.
(4) The method according to item (3), wherein the method further comprises a step of preparing a substance which can optimally bind to the receptor, by screening a length and a structure of a linker for linking the substance which can optimally bind to the first binding site and the substance which can optimally bind to the second binding site.
(5) A method for screening a substance which can bind to a receptor having n (n indicates an integer 2 or more) binding sites consisting of a first binding site to a n^{th} binding site, by monomolecular fluorescent analysis, comprising:
   a step of searching a substance which can bind to a first binding site; and
   a step of repeating a step of searching a substance which can bind to a X^{th} binding site (n-1) times sequentially from X = 2 to X = n, under condition where each substance which can bind to (X-1) binding sites consisting of a first binding site to a (X-1)^{th} binding site is bound to the receptor.
(6) A method for screening a substance which can optimally bind to a receptor having n (n indicates an integer of 2 or more) binding sites consisting of a first binding site to a n^{th} binding site, by monomolecular fluorescent analysis, comprising:
   a first step of searching a substance which can bind to a first binding site;
   a second step of performing optimization of a structure of the substance which can bind to the first binding site obtained by the above first step;
   a third step of searching a substance which can bind to a X^{th} binding site, under condition where each substance which can bind to (X-1) binding sites consisting of a first binding site to a (X-1)^{th} binding site is optimally bound to the receptor; and
   a fourth step of performing optimization of a structure of the substance which can bind to the X^{th} binding site obtained by the above third step,
   wherein the third step and the forth step are repeated (n-1) times sequentially from X = 2 to X = n.
(7) The method according to item (6), wherein the method further comprises a step of preparing a substance which can optimally bind to the receptor, by screening a length and a structure of each linker for linking n substances, respectively, consisting of the substance which can optimally bind to the first biding site to the substance which can optimally bind to the n^{th} binding site.
(8) The method according to any one of items (1) to (7), wherein the substance which can bind to the receptor is a target drug in drug design.
(9) The method according to any one of items (1) to (8), wherein the substance which can bind to the receptor is labeled with a substance which can be optically detected.

### Brief Description of Drawings

FIG. 1 is a view showing a cycle of drug design.
FIG. 2 is a view showing outline of SAR by NMR.
FIG. 3 is a view explaining outline of Fluorescence Intensity Distribution Analysis (FIDA).
FIG. 4 is a view showing outline of an example in which the screening method of the present invention is applied to drug design screening.
FIG. 5 is a view showing one example of a fluorescent correlation analysis apparatus used in the screening method of the present invention.

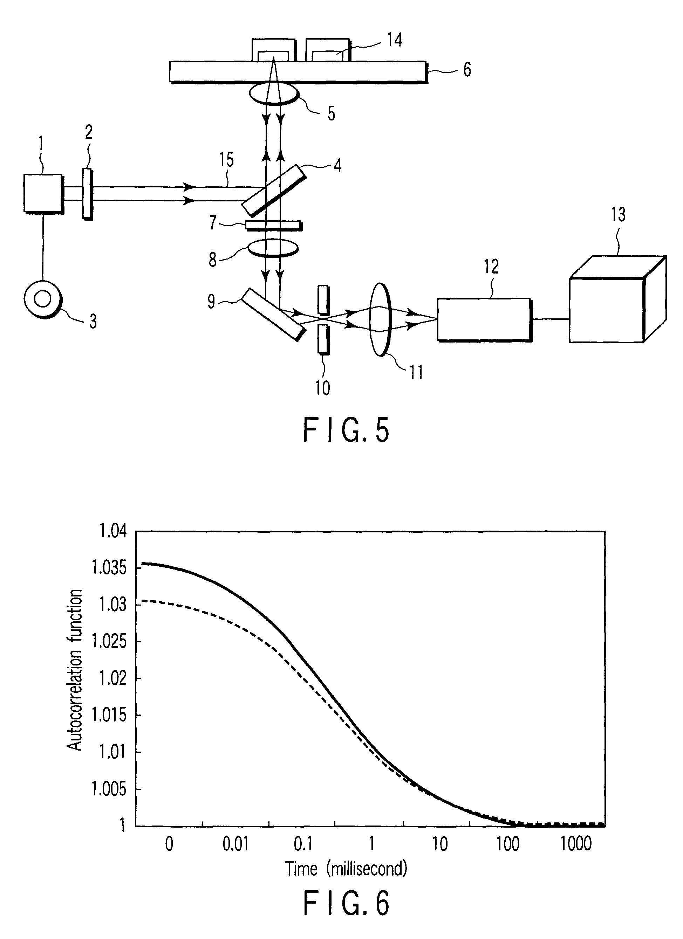

   In FIG. 5, reference numerals 1 to 15 denote the following:
   1... laser light source, 2... light intensity regulating means (ND filter), 3... light attenuation rate selecting apparatus (ND filter changer), 4... dichroic mirror, 5... objective lens, 6... stage, 7... filter, 8... tube lens, 9... reflection mirror, 10... pinhole, 11... lens, 12... light detector (avalanche photodiode), 13... fluorescent intensity recording means (computer), 14... sample, 15... light beam.

   FIG. 6 is a view showing data of monomolecular fluorescent analysis (autocorrelation function) of a substance which can bind to a receptor.

### Best Mode for Carrying Out the Invention

### <Monomolecular fluorescent analysis technique>

First, monomolecular fluorescent analysis which is an analysis technique used in a screening method of the present invention will be explained.

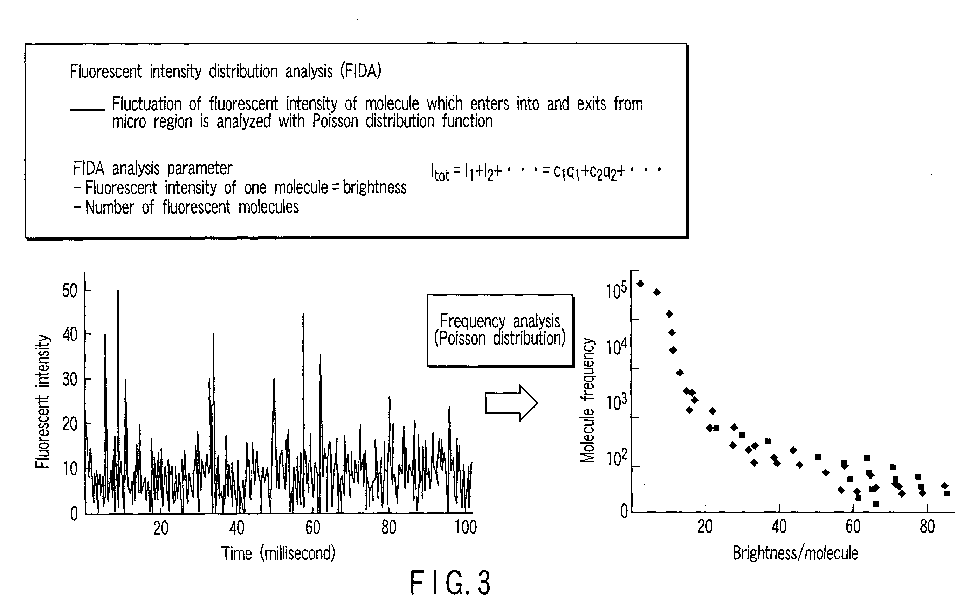

Monomolecular fluorescent analysis is technique of measuring fluctuation movement of a fluorescent molecule which enters into and exits from a micro confocal region, and analyzing the obtained data by a function. This technique includes (1) analysis by Fluorescence Correlation Spectroscopy, (2) Fluorescence Intensity Distribution Analysis, and (3) Fluorescence Intensity Multiple Distribution Analysis performing these analyses simultaneously. Each of them will be explained below.
(1) Fluorescence Correlation Spectroscopy (hereinafter, also referred to as FCS) is a technique of measuring fluctuation movement of a fluorescence labeled target molecule in a medium, and correctly measuring a micromovement of individual target molecule by using Autocorrelation function (Reference Publication: D. Magde and E. Elson, "Fluorescence correlation spectroscopy. II. An experimental realization", Biopolymers 1974 13(1) 29-61).
   FCS is performed by analyzing a diffusion time from fluctuation of a fluorescent intensity by capturing Blownian movement of a fluorescent molecule in a micro-region in a solution with a laser confocal microscope, and determining a physical amount (the number and size of molecules). Analysis by FCS capturing molecular fluctuation in such the micro-region is effective for detecting intermolecular interaction at a high sensitivity and specifically.
   Principle of detection by FCS will be explained in more detail. In FCS, a fluorescent signal generated from a micro field region in a sample is detected and is quantified with a microscope. Then, a fluorescence labeled target molecule in a medium is always moved (Blownian movement). Therefore, a detected fluorescent intensity is changed depending on a frequency of entrance of a target molecule into a micro field region, and a time during which the molecule stays in the region.
   For example, when an apparent molecular weight is increased by occurrence of dimerization of molecules, movement of a target molecule is slowed down, and the apparent molecular number is decreased. As a result, a frequency of entrance of a target molecule into a micro field region is reduced, and an observed fluorescent intensity is changed. By monitoring such the change in a fluorescent intensity, a change in an apparent molecular weight of a target molecule can be traced.
(2) Fluorescence Intensity Distribution Analysis (hereinafter, also referred to as FIDA) is disclosed in the references: P Kask, et al; PNAS 23, 96, 13756-13761, 1999, and WO 98/16814. FIDA is a technique of irradiating a sample with laser, measuring the excitation light of a fluorescent molecule emitted from a sample with APD (high sensitive photodetector), and analyzing the measured fluorescent signal with double Poisson distribution function regarding photon count resolved per very short time of 40 microseconds, followed by statistical analysis. By FIDA, a fluorescent intensity per molecule (brightness; qn) and the number of fluorescent molecule (cn) are calculated (see FIG. 3). Even when there is plurality of molecule types, they can be discriminated by distribution analysis, and a numerical value obtained by multiplying brightness and the molecule number regarding each molecule type can be calculated as a total fluorescent amount. FIG. 3 shows an example in which fluctuation of a fluorescent intensity of a fluorescent molecule which enters into and exits from a micro confocal region was analyzed by a Poisson distribution function.
(3) Fluorescence Intensity Multiple Distribution Analysis (hereinafter, also referred to as FIMDA) performs FCS analysis and FIDA simultaneously. Detailed contents are disclosed in the Reference: (K Palo, Biophysical Journal, 79, 2858-2866, 2000). According to FIMDA, data regarding a translational diffusion time of a fluorescent molecule, the number of molecules, and a fluorescent intensity per molecule (brightness) can be acquired simultaneously. For this reason, not only a size of a molecule can be determined, but also a change in a size of a molecule which is around 5-fold can be discriminated based on a difference in brightness, which could not be discriminated by FCS.

### <Screening method of the present invention>

Using the aforementioned monomolecular fluorescent analysis technique, a screening method of the present invention can be performed. The method of the present invention will be explained in detail below.

The screening method of the present invention is a method for screening a substance which can optimally bind to a receptor, by monomolecular fluorescent analysis, comprising:
a step of searching a substance which can bind to a receptor; and
a step of optimizing a structure of the substance which can bind to the receptor obtained by the above step.

### (Receptor)

A receptor used in the method of the present invention may be an arbitrary receptor, and is not particularly limited as far as it receives a ligand *in vivo*, and causes various response reactions *in vivo* by the received signal. Particularly, a receptor in which various response reactions *in vivo* caused by the received signal are associated with a disease, is preferably used. A size of a receptor used in the method of the present invention is not particularly limited, but may be various sizes of from 0.4 kDa to 800 kDa. Examples of a receptor used in the method of the present invention include GPCR (G protein-coupled receptor).

A receptor obtained by any preparing method may be used. For example, the receptor may be obtained by *in vitro* expression by the known genetic engineering procedure using a gene coding the receptor may be utilized. Alternatively, a crudely purified material may be obtained by collecting a fraction containing a receptor from *in vivo.*

### (Step of searching a substance which can bind to a receptor)

In the present invention, a substance which can bind to a receptor is searched among an arbitrary substance which is suspected of binding to the receptor and causing a response reaction *in vivo* (hereinafter, also referred to as candidate substance). In the present invention, the candidate substance includes any of an organic compound and an inorganic compound, and examples thereof include molecules constituting a living organism such as a protein, a glycoprotein, a lipid and an amino acid, and chemical substances such as a hormone, an autacoid, an ion, and a metal.

In the present invention, at least one of the candidate substance and the receptor must be labeled for monomolecular fluorescent analysis. As a representative pattern of labeling, the cases (A) to (C) are listed, but the present invention is not limited to them.
(A) Case where a candidate substance is labeled
   In this case, since a free candidate substance and a candidate substance bound to a receptor are clearly different in a molecule size, they can be discriminated by monomolecular fluorescent analysis.
(B) Case where a receptor is labeled
   In the case where a receptor receives a ligand and then a change in a size of a receptor molecule such as dimerization of receptor molecules is caused, a receptor can be labeled. That is, such reception of a candidate substance by a receptor can be discriminated by a change in a size of a receptor molecule, by monomolecular fluorescent analysis.
(C) Case where both of a candidate substance and a receptor are labeled with the same labeling substance
   When a candidate substance is bound to a receptor, fluorescent intensity per receptor molecule becomes 2-fold. Thereby, it can be discriminated by a change in a fluorescent intensity, by monomolecular fluorescent analysis.

As a marker substance for labeling, an arbitrary marker substance can be used as far as it is a substance emitting a signal which can be optically traced, that is, a substance which can be detected by light. Various pigments which generate a quantifiable stable signal are preferable, and fluorescent pigments by which the presence of a small substance can be separately measured are particularly preferable. Preferably, an arbitrary fluorescent substance which emits detectable fluorescent light can be used. For example, various fluorescent pigments such as fluorescein isothiocyanate (FITC), rhodamine, TAMRA, Cy3 (Amersham Pharmacia), and Cy5 (Amersham Pharmacia) can be used. Fluorescent labeling can be performed by the known procedure.

A step of searching a substance which can bind to a receptor is performed by placing a receptor and a candidate substance in a prescribed solution. The prescribed solution can be a solution in which a receptor and an inherent ligand of the receptor can be bound. For example, a physiological saline or a phosphate buffer can be used.

By maintaining a receptor and a candidate substance under the prescribed condition in this prescribed solution, only a substance which can bind to a receptor among candidate substances causes a binding reaction. Herein, the prescribed condition (temperature, pH, reaction time etc.) can be appropriately set depending on a kind of a receptor.

An amount of a candidate substance to be added to a reaction solution is suitably such an amount that the final concentration becomes around 0.01 to 100 nM, further preferably around 0.1 to 50 nM. In addition, an amount of a receptor to be added is suitably such an amount that the final concentration becomes around 0.01 nM to 10 µM, further preferably around 0.1 to 50 nM.

As one example of a reaction, a reaction can be performed by mixing each 50 µL of a solution containing 10 nmol/L of a receptor (FK506-binding protein) in a physiological buffer and a solution containing 1 to 100 nmol/L of a candidate substance labeled with 5-TAMRA in a physiological buffer, and incubating them at room temperature for 15 to 30 minutes, as mentioned in Examples described later.

For incubation, a reaction solution containing a set of reaction components in the suspended state in the prescribed solution can be retained in an appropriate liquid retaining means such as a test tube, a well, a cuvette, a groove, a tube, a plate, and a porous medium. Herein, a shape, a material and a size of the liquid retaining means are preferably selected so that a part or all of various detection steps such as dispensing, stirring, incubation, measurement, and conveyance are rapidly performed. For example, since measurement by monomolecular fluorescent analysis uses an extremely fine region at an optical focus level as a measurement place, the means can be a very small-type of liquid accommodating means. Particularly, in detection by optical measurement, the liquid retaining means preferably has an opening part for entrance and/or exit of a measuring beam so that the light beam for measurement is directly associated with reaction components as much as possible.

A presence ratio of a candidate substance bound to a receptor and a candidate substance free in the reaction solution can be measured by monomolecular fluorescent analysis based on a difference in respective molecular weights. Thereby, easiness (affinity) of binding of a candidate substance to a receptor can be obtained as a dissociation constant.

When a substance which can bind to a receptor is found out among candidate substances, a structure of the substance is then optimized. In the case where the screening method of the present invention is applied to drug design screening, all substances which can bind to a receptor can be called "hit compound". In addition, among "hit compounds", compounds which have a strong binding force to a receptor and are transferred to a later step of optimizing a structure can be called "lead compound".

Hereinafter, for convenience, a substance which is a base for optimizing a structure is referred to as "lead compound", but it goes without saying that the screening method of the present invention is not limited to application of the drug design screening.

Optimization of a structure refers to preparation of compounds (e.g. the number of compounds: 5 to 20) having various structures by modifying/altering a side chain of a lead compound, investigation of ability of these various compounds to bind to a receptor, and selection of an optimal compound among them. Optimization in the present invention is operation of selecting a compound having the highest binding force among various compounds prepared by modification/alteration.

Examples of modification/alteration of a side chain include alteration of an amide group contained in a compound into a carboxyl group or a hydroxyl group. An example of actual alteration of a side chain of a compound for structural optimization will be mentioned in Examples described later, and outline thereof will be shown in Chemical Formula described later. However, the modification/alteration is not limited to this example, arbitrary alteration/modification of a side chain is possible, and a person skilled in the art can appropriately perform the alteration/modification.

Ability of a compound to bind to a receptor can be obtained by monomolecular fluorescent analysis based on a change in a molecular weight or a change in a fluorescent intensity per receptor molecule, as described above.

### (Preferable embodiment)

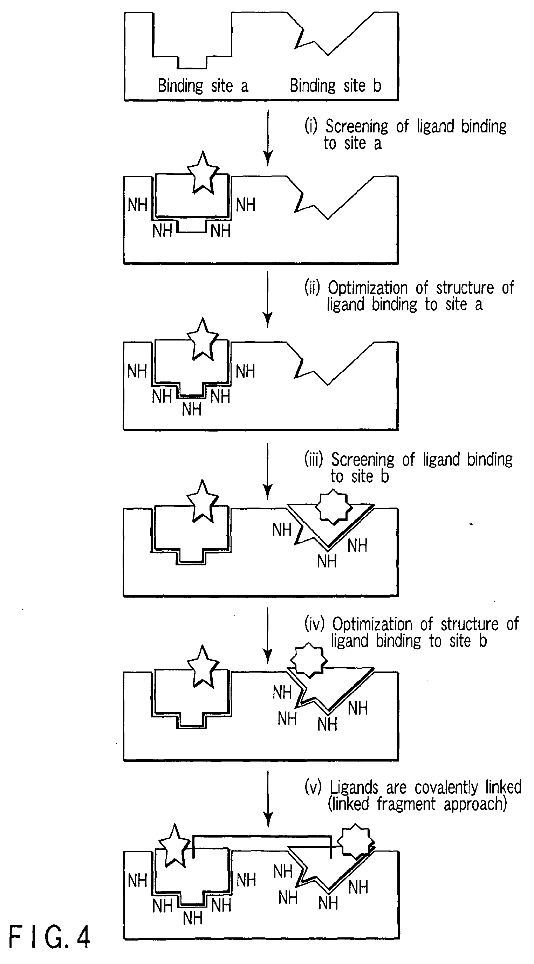

As a preferable embodiment, an example of application of the screening method of the present invention to drug design screening will be explained below. Outline of a preferable embodiment is shown in FIG. 4. However, it goes without saying that the present invention is not limited to the following embodiment.

That is, a preferable embodiment of the present invention is,
a method for screening a substance which can optimally bind to a receptor having a first binding site and a second binding site, comprising:
(i) a step of searching a substance which can bind to the first binding site;
(ii) a step of optimizing a structure of the substance which can bind to the first binding site, obtained by the aforementioned step;
(iii) a step of searching a substance which can bind to the second binding site, under condition where the substance which can optimally bind to the first binding site obtained by the aforementioned step is bound to the receptor;
(iv) a step of optimizing a structure of the substance which can bind to the second binding site obtained by the aforementioned step; and
(v) a step of preparing a substance which can optimally bind to the receptor, by screening a length and a structure of a linker for linking the substance which can optimally bind to the first binding site and the substance which can optimally bind to the second binding site.

The aforementioned steps (i) to (v) correspond to (i) to (v) of FIG. 4. These steps will be explained below in an order of a step.

First, a receptor used herein has a site a to which a substance which can bind to a receptor (also referred to as substance a) binds and, further, has a binding site b to which another substance (also referred to as substance b) binds in addition to the binding site a. It is generally preferable that, in drug design screening, the binding site a and the binding site b are situated in a vicinity. Herein, the vicinity means that the binding site a and the binding site b are sterically situated so that they can bind sterically with a linker.

It is well-known to a person skilled in the art that such screening of a substance which can bind to each of two sites is an effective procedure in drug design screening upon development of a highly effective drug.
"(i) Screening of a ligand which binds to a site a"
   In a step (i), a substance (substance a) which can bind to one binding site (binding site a in FIG. 4) among two binding sites is searched. For this step, see the aforementioned explanation.
"(ii) Optimization of a structure of a ligand which binds to a site a"
   In a step (ii), optimization of a structure of the substance which can bind to the binding site a obtained by the step (i) is performed. Also for this step, see the aforementioned explanation.
"(iii) Screening of a ligand which binds to a site b"
   In a step (iii), a substance (substance b) which can bind to another binding site b is searched, under condition where the substance which can optimally bind to the binding site a, obtained by the step (ii), is bound to the receptor. A step of (iii) can be performed as in the step of (i).
   Herein, it is preferable that the substance a and the substance b are discriminably labeled with two kinds of fluorescent substances having different excitation wavelengths, respectively, as shown in FIG. 4. Thereby, binding of the substance a to the receptor, and binding of the substance b to the receptor can be discriminably recognized, respectively. As a labeling substance, any labeling substance may be used as far as it generates a signal which can be optically traced, as described above.
   In addition, "under condition where a substance which can optimally bind to a binding site a is bound to the receptor" refers to the condition under which a substance which can optimally bind to a binding site a is mixed with a receptor in a solution, and a substance bound to a receptor and a free substance not bound thereto are present in the equilibrium state.
"(iv) Optimization of a structure of a ligand which binds to a site b"
   In a step (iv), optimization of a structure of a substance which can bind to a binding site b, obtained by the step (iii) is performed. The step (iv) can be performed as in the step of (ii).
"(v) Ligands are covalently linked (linked fragment approach)"
   In a step of (v), a length and a structure of a linker for linking a substance (substance A) which can optimally bind to a binding site a and a substance (substance B) which can optimally bind to a binding site b are screened. Thereby, a substance which can optimally bind to the receptor can be prepared.
   The linker is not particularly limited as far as it can link the substance A and the substance B. For example, as mentioned in Examples described later, a linker in which various numbers of methylene groups (-CH₂- group) are connected, can be used.

### <Case where a receptor has two or more binding sites>

Also in the case where a receptor molecule has two or more binding sites, a substance which can bind to the receptor molecule can be screened similarly. That is, a method of screening a substance which can bind to a receptor having two or more binding sites is preferably as described below.

That is, the method is a method of screening a substance which can optimally bind to a receptor having n (n indicates an integer of 2 or more) binding sites consisting of a first binding site to n^{th} binding site, by monomolecular fluorescent analysis, comprising:
a first step of searching a substance which can bind to a first binding site;
a second step of optimizing a structure of the substance which can bind to the first binding site, obtained by the first step;
a third step of searching a substance which can bind to a X^{th} binding site, under condition where each substance which can bind to (X-1) binding sites consisting of a first binding site to a (X-1)^{th} binding site is optimally bound to the receptor, respectively;
a fourth step of optimizing a structure of the substance which can bind to the X^{th} binding site, obtained by the third step,
wherein the third step and the fourth step are repeated sequentially (n-1) times from X = 2 to X = n; and
a fifth step of preparing a substance which can optimally bind to the receptor, by screening a length and a structure of each linker for linking n substances consisting of a substance which can optimally bind to the first binding site to a substance which can optimally bind to the n^{th} binding site, respectively.

Also in the case where a receptor has two or more binding sites, screening a substance which can bind to the receptor is performed in the same way as the method for screening a substance which can bind to a receptor having two binding sites, and see explanation thereof. Generally, in the case of drug design, a receptor having 2 to 5 binding sites is supposed in many cases.

### <Apparatus for performing monomolecular fluorescent analysis>

An example of an apparatus for performing monomolecular fluorescent analysis (hereinafter, also referred to as fluorescent correlation analysis apparatus) will be explained below by referring to FIG. 5.

As shown in FIG. 5, the fluorescent correlation analysis apparatus is equipped with a laser light source 1; a light intensity regulating means (herein, ND filter) 2 for attenuating an intensity of a light beam 15 from the laser light source 1; a light attenuation selecting apparatus (herein, ND filter changer) 3 for setting an appropriate light intensity regulating means 2; a stage 6 on which a sample 14 containing a fluorescent molecule is placed; optical systems 4 and 5 for concentrating a light beam 15 from the laser light source 1 on the sample 14 to form a confocal region; optical systems 7 to 11 for concentrating the fluorescence emitted from the sample 14; a light detector 12 for detecting the concentrated fluorescence; and a fluorescent intensity recording means 13 for recording a change in a fluorescent intensity. As described, the fluorescent correlation analysis apparatus utilizes a confocal laser microscope.

In FIG. 5, laser radiated from the laser light source 1 may be any of argon ion laser, helium-neon laser, krypton, and helium-cadmium. In FIG. 5, optical systems 4 and 5 for concentrating a light beam 15 from the laser light source on the sample to form a confocal region specifically mean a dichroic mirror 4, and an objective lens 5. The light beam 15 from the laser light source 1 proceeds through a route as shown by an arrow in FIG. 5. That is, an intensity of the light beam 15 from the laser light source is first attenuated according to an attenuation degree of the fluorescent intensity regulating means (herein ND filter) 2, then, the light beam 15 is refracted in a direction of a stage 90 degree relative to incident light with the dichroic mirror 4, and irradiated on a sample on the stage 6 through the objective lens 5. In this way, the light beam is concentrated on the sample at a fine one spot to form a confocal region.

In FIG. 5, optical systems 7 to 11 for concentrating the fluorescent light radiated from a fluorescent molecule in a confocal region specifically mean a filter 7, a tube lens 8, a reflection mirror 9, a pinhole 10, and a lens 11. The fluorescence radiated from a florescent molecule proceeds through a route shown with an arrow in FIG. 5. That is, the fluorescence radiated from a florescent molecule is first passed through the dichroic mirror 4 in a light progression direction, refracted with the reflection mirror 9 via the filter 7 and the tube lens 8 to form an image on the pinhole 10, passed through the lens 11, and concentrated on a light detector 12.

The light detector (herein, avalanche photodiode) 12 for detecting the concentrated fluorescence converts the received light signal into an electric signal, and transmits it to a fluorescent intensity recording means (herein, computer) 13.

The fluorescent intensity recording means 13 for recording a change in a fluorescent intensity performs recording and analysis of transmitted fluorescent intensity data. Specifically, by analysis of the fluorescent intensity data, an autocorrelation function is set. Increase in a molecular weight due to binding of a fluorescent molecule to a receptor, and decrease in the number of free fluorescent molecules can be detected by a change in an autocorrelation function.

An apparatus for performing monomolecular fluorescent analysis (fluorescence correlation analysis apparatus) is not limited to the example shown in FIG. 5. For example, as shown in FIG. 4, when a substance a which binds to a binding site a and a substance b which binds to a binding site b are discriminably labeled with two kinds of fluorescent substances having different excitation wavelengths, and thereby a substance which can bind to each binding site is screened, it is necessary that the fluorescence correlation analysis apparatus has two kinds of laser light sources for exciting respective fluorescent substances, and two kinds of light detectors for detecting the lights radiated from respective fluorescent substances. The light detector may be an apparatus comprising a photomultiplier tube in addition to APD (avalanche photodiode).

### <Regarding effect of the present invention>

As described above, the screening method using a monomolecular fluorescent analysis method of the present invention has the following advantages as compared with the conventional structure-activity relationship using NMR as an analysis procedure (SAR by NMR).
(1) Although a NMR apparatus is very expensive such as 200 to 300 million yen, the fluorescent correlation analysis apparatus used in the present invention has a low cost (around a few tens million) and a low measuring cost, as compared therewith.
(2) Regarding an operating method, NMR needs synthesis and handling of a radioactive compound, and also knowledge of NMR is required, but in a fluorescent correlation analysis apparatus, special professional ability is not essential, and the operation is simple.
(3) As an amount of a sample solution used in the method of the present invention, a few tens microliter is sufficient. In addition, the screening method has an advantage that, even when a concentration of a sample in a sample solution is low, an assay can be performed at a high sensitivity.
(4) As a receptor molecule which can be used in the method of the present invention, in addition to a molecule having a molecular weight of up to 20 kDa which can be measured by a method of NMR, a large molecule of 100 kDa or more can be used. Further, in NMR measurement, it is necessary that a receptor has a high purity, but in the present invention, it is not necessary to purify a receptor molecule used.
   In addition, in NMR, since analysis of a structure of a large molecule becomes complicated, it is necessary to prepare a few kinds of molecules by using different radioactive labels every atom, but in a fluorescent correlation analysis apparatus, reception of a substance by a receptor can be easily analyzed by fluorescent labeling.
   In addition, in the case of a molecule usually having a size of 50 kDa or more like a receptor molecule, in NMR, separation by a genetic engineering procedure every domain is necessary, but in a fluorescent correlation analysis apparatus, such the DNA recombinant step is not necessary. Therefore, the method of the present invention exerts particularly excellent effect in screening a drug having a binding site spanning a plurality of domains.
(5) Even a sample having a complicated structure such as a lipid and a glycoprotein which were unsuitable for NMR measurement can be measured in the present invention using monomolecular fluorescent analysis technique.
(6) In NMR, unless structures of a receptor and a substance binding thereto are already known, they can not be used in measurement, but in the screening method of the present invention, even when respective structures are not known, they can be used. Thereby, in the screening method of the present invention, a substance in which a side chain of a compound is randomly altered can be used as a candidate substance.
(7) In NMR, whether a receptor and a test substance can be bound or not can be detected, but a strength of a binding force can not be investigated, but in the present invention, by applying monomolecular fluorescent analysis technique, a presence ratio of a test substance bound to a receptor and a test substance in the free state can be obtained. Thereby, a binding force (affinity) of each substance and a receptor can be determined.
(8) In NMR, a very long measuring time (one month in some cases) is necessary, but in the present invention, by applying monomolecular fluorescent analysis technique, measurement in a short time is possible. Generally, a few seconds to a few tens seconds is sufficient for a measurement. Thereby, the method of the present invention is suitable for high throughput screening.
   To summarize the foregoing, according to the screening method of the present invention, all the problems of the conventional procedure using NMR can be solved. Inter alia, in the method of the present invention, when interaction between a compound and a biomolecule (e.g. receptor) is seen, interaction at a molecular level can be measured more rapidly and at a low cost. In addition, in the conventional structure-activity relationship by NMR, only a small domain such as a protein of 20 kDa can be analyzed, but in the method of the present invention, a protein of 100 kDa or more can be also analyzed.

### <Examples>

Regarding search of an inhibitor of a FK506-binding protein (FKBP), screening was performed with a fluorescent correlation analysis apparatus. Outline thereof is shown in the following Chemical Formula.

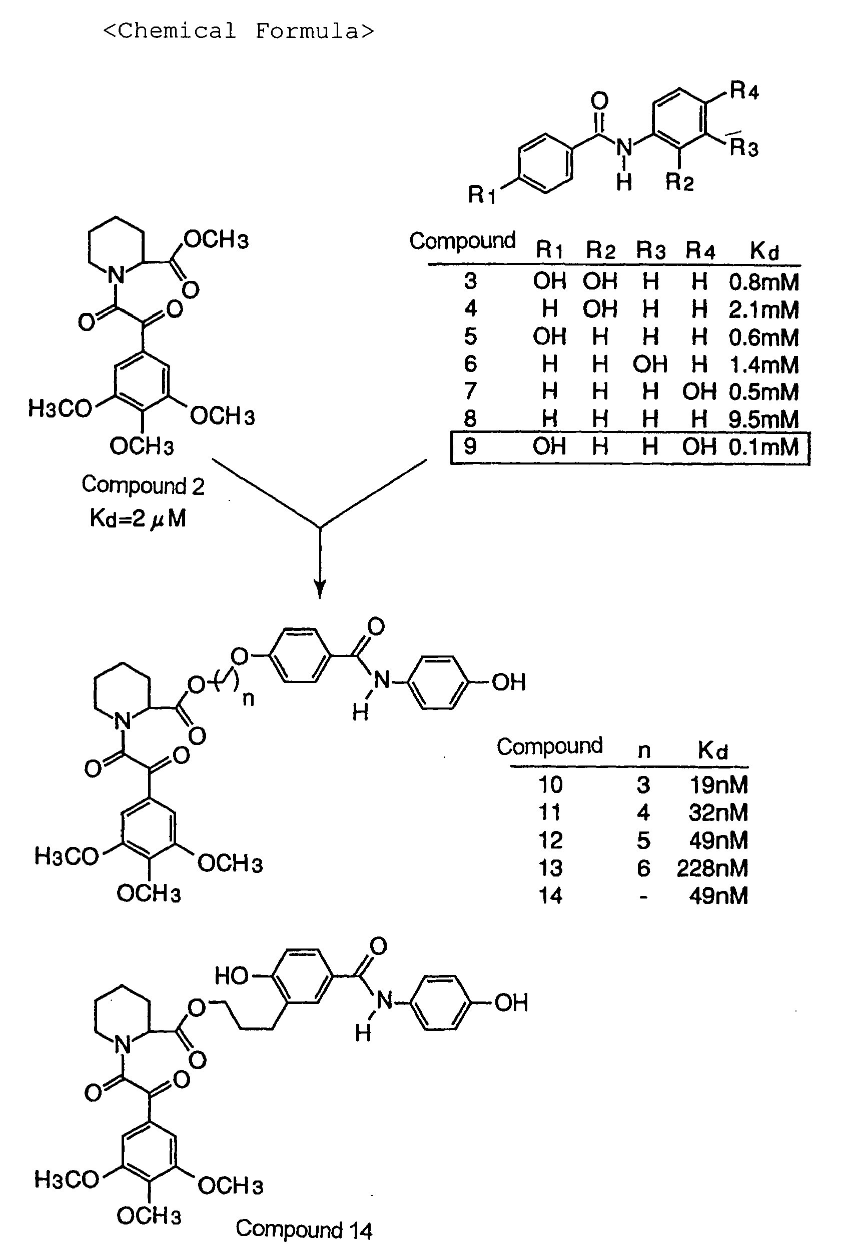

The Chemical Formula shows outline of an example in which an inhibitor of a FK506-binding protein (FKBP) was searched using the screening method of the present invention.

FKBP, when binds to FK506 (Tacrolimus) which is a strong immunosuppressant, blocks activation of a T cell by inhibiting calcineurin which is serine/threonine phosphatase.

As a target protein, FKBP was used, and a concentration thereof was adjusted to 10 nmol/L in a physiological buffer. A library of a plurality of compounds (also referred to as first compound library) was labeled with a fluorescent substance, 5-TAMRA (5-Carboxytetramethylrhodamine), and was adjusted to three concentrations (1, 10, and 100 nmol/L) (hereinafter, such fluorescence-labeled compound is referred to as first fluorescence-labeled compound). The target protein FKBP and the first fluorescence-labeled compound having any of three concentrations (1, 10, and 100 nmol/L) were mixed at the same amount of 50 µL. The mixture was incubated at room temperature for 15 to 30 minutes, placed in a 96-well glass bottom microplate, and measured with a fluorescent correlation analysis apparatus.

As laser, helium-neon laser 543 nm was used. Measurement was performed consecutively five times at a measuring time of 10 seconds per sample, and an average was adopted as data. As a measuring parameter, a translational diffusion time and a molecular concentration were calculated, and affinity of each compound was studied by a Kd value (dissociation constant).
(i) a first fluorescence-labeled compound alone, and (ii) a target protein (FKBP) which had interacted with the compound were discriminated by a translational diffusion time, and thereby discriminated as two kinds of molecular species. (i) The first fluorescence-labeled compound has a molecular weight of 700 to 1700, and (ii) the target protein (FKBP) which had interacted with the compound has a molecular weight of about 30 kDa. Therefore, a translational diffusion time obtained by a monomolecular fluorescent correlation analysis apparatus was about 60 µ seconds in (i) the first fluorescence-labeled compound, and about 240 µ seconds in (ii) the target protein (FKBP) which had interacted with the compound. By a difference in such translational diffusion time, (i) and (ii) could be discriminated in the same sample, and a molecular concentration of each of them could be calculated.

Among a plurality of first fluorescence-labeled compounds, a few kinds showed affinity with the target protein (FKBP). The compounds which showed affinity were re-screened, and a trimethoxyphenylpipecolic acid derivative (Compound 2 shown in the Chemical Formula) showed sufficiently high affinity (Kd = 2 µM).

Then, a mixture solution of a Compound 2 and a target protein FKBP was prepared. In the mixture solution, the Compound 2 interacts with FKBP and, thereby, a Compound 2 which is bound to FKBP and a Compound 2 which is in a free state are present in admixture with each other in the equilibrium state. Using such the mixture solution, a compound which binds to FKBP which had interacted with Compound 2, in a vicinity of the Compound 2, was further screened among a group of a few kinds of compounds (also referred to as second compound library).

The second compound library was labeled with Cy5 (Amersham) as a fluorescent substance (hereinafter, such a fluorescence-labeled compound is referred to as second fluorescence-labeled compound). In a fluorescent correlation analysis apparatus, helium-neon laser 633 nm was irradiated for measurement. The second fluorescence-labeled compound uses a different excitation wavelength from that of the aforementioned first fluorescence-labeled compound, upon detection. For this reason, when a wavelength exciting only a second fluorescence-labeled compound is irradiated, only a sample with which two kinds of fluorescence-labeled compounds interacted can be screened.

A translational diffusion time measured by a fluorescent correlation analysis apparatus is about 60 µ seconds in (i) the second fluorescence-labeled compound, and about 240 µ seconds in (ii) a target protein (FKBP) with which both of the first fluorescence-labeled compound and the second fluorescence-labeled compound interacted.

Since the analysis can be performed in around a few second to 1 minute per sample, samples could be measured considerable rapidly. As a result, a compound having Kd = 0.8 mM (Compound 3 shown in the Chemical Formula) was hit.

Data (autocorrelation function) of the hit Compound 3 measured by a fluorescent correlation analysis apparatus are shown in FIG. 6. The data are analysis data of an autocorrelation function for calculating a translational diffusion time of a complex obtained by interaction of a target protein and two kinds of compounds (i.e., a complex of a target protein, a Compound 2 and a Compound 3). An abscissa axis indicates a time (millisecond) and an ordinate axis indicates an autocorrelation function. A dotted line indicates data detected with laser of a wavelength 543 nm and a solid line indicates data detected with laser of 633 nm.

Thereby, using about 240 µ seconds of a translational diffusion time of the complex obtained by interaction with two kinds of compounds, a concentration of each molecular species (i.e., a concentration of a fluorescence-labeled compound in a free state and a concentration of the complex obtained by interaction) was calculated. As a result, a Kd value (dissociation constant) which is to be an index of affinity was obtained.

Further, Compounds 3 to 9 in which a side chain of the Compound 3 was altered were prepared, and Kd values (dissociation constant) of respective compounds were obtained. It was seen that a Compound 9 most stably binds to a target protein.

By covalently binding the Compound 2 (Kd = 2 µM) and the Compound 9 (Kd = 100 µM) with a suitable linker, a more stabilized complex can be formed. From a chemical structure of the complex, it is seen that a methyl ester group of the Compound 2 and a benzoyl group of the Compound 9 are near in the state of the complex. For this reason, five kinds of compounds 10 to 14 were synthesized with a linker consisting of a methylene group of a different length. As a result, all of them showed a dissociation constant at nM order. Among these five kinds of compounds, a compound having highest affinity for FKBP is a Compound 10, and it was seen that Kd = 19 nM.

### Industrial Applicability

According to the present invention, a method for screening a substance which can bind to a receptor is provided. Particularly, the present invention is useful in structure-activity correlation analysis for effectively finding out a ligand of a receptor which is to be a novel drug design target and performing drug molecule design.

## Claims

1. A method for screening a substance which can optimally bind to a receptor, by monomolecular fluorescent analysis, **characterized by** comprising:
a step of searching a substance which can bind to a receptor; and
a step of performing optimization of a structure of the substance which can bind to the receptor obtained by the above step.

2. A method for screening a substance which can bind to a receptor having a first binding site and a second binding site, by monomolecular fluorescent analysis, **characterized by** comprising:
a step of searching a substance which can bind to the first binding site; and
a step of searching a substance which can bind to the second binding site, under condition where the substance which can bind to the first binding site obtained by the above step is bound to the receptor.

3. A method for screening a substance which can optimally bind to a receptor having a first binding site and a second binding site, by monomolecular fluorescent analysis, **characterized by** comprising:
a step of searching a substance which can bind to a first binding site;
a step of performing optimization of a structure of the substance which can bind to the first binding site obtained by the above step;
a step of searching a substance which can bind to the second binding site, under condition where the substance which can optimally bind to the first binding site obtained by the above step is bound to the receptor; and
a step of performing optimization of a structure of the substance which can bind to the second binding site obtained by the above step.

4. The method according to claim 3, **characterized in that** the method further comprises a step of preparing a substance which can optimally bind to the receptor, by screening a length and a structure of a linker for linking the substance which can optimally bind to the first binding site and the substance which can optimally bind to the second binding site.

5. A method for screening a substance which can bind to a receptor having n (n indicates an integer 2 or more) binding sites consisting of a first binding site to a n^{th} binding site, by monomolecular fluorescent analysis, **characterized by** comprising:
a step of searching a substance which can bind to a first binding site; and
a step of repeating a step of searching a substance which can bind to a X^{th} binding site (n-1) times sequentially from X = 2 to X = n, under condition where each substance which can bind to (X-1) binding sites consisting of a first binding site to a (X-1)^{th} binding site is bound to the receptor.

6. A method for screening a substance which can optimally bind to a receptor having n (n indicates an integer of 2 or more) binding sites consisting of a first binding site to a n^{th} binding site, by monomolecular fluorescent analysis, **characterized by** comprising:
a first step of searching a substance which can bind to a first binding site;
a second step of performing optimization of a structure of the substance which can bind to the first binding site obtained by the above first step;
a third step of searching a substance which can bind to a X^{th} binding site, under condition where each substance which can bind to (X-1) binding sites consisting of a first binding site to a (X-1)^{th} binding site is optimally bound to the receptor; and
a fourth step of performing optimization of a structure of the substance which can bind to the X^{th} binding site obtained by the above third step,
wherein the third step and the forth step are repeated (n-1) times sequentially from X = 2 to X = n.

7. The method according to claim 6, **characterized in that** the method further comprises a step of preparing a substance which can optimally bind to the receptor, by screening a length and a structure of each linker for linking n substances, respectively, consisting of the substance which can optimally bind to the first biding site to the substance which can optimally bind to the n^{th} binding site.

8. The method according to any one of claims 1 to 7, **characterized in that** the substance which can bind to the receptor is a target drug in drug design.

9. The method according to claim 1, **characterized in that** the substance which can bind to the receptor is labeled with a substance which can be optically detected.
